# EUROPEAN PATENT APPLICATION

(11) **EP 4 503 040 A1**
(43) Date of publication of application: **05.02.2025**
(21) Application number: 23189718.2
(22) Date of filing: 04.08.2023
(51) Int. Cl.: G16C 10/00

(54) **METHOD AND SYSTEM FOR ESTIMATING A GROUND STATE ENERGY OF A QUANTUM CHEMICAL SYSTEM**

(71) Applicant: VOLKSWAGEN AG, 38440 Wolfsburg (DE); Xanadu Quantum Technologies Inc., Toronto ON M5G 2C8 (CA)
(72) Inventor: Arrazola Mantilla, Juan Miguel, Toronto, ON M6N 0B7 (CA); Hejazi, Kasra, Los Angeles, 90025 (US); Shokrian Zini, Modjtaba, Toronto, ON M4W 3G9 (CA); Fomichev, Stepan, Toronto, ON M4S 0B8 (CA); Kiser, Matthew Royal, 81677 München (DE)
(74) Representative: Zucker, Volker

(57) **Abstract**

The invention relates to a method for estimating a ground state energy (10) of a quantum chemical system, the method comprising: classically computing states (4) close to the ground state of the quantum chemical system, assessing the quality of the classically computed states (4) based on the energy distribution of each state (4), selecting a state (4) based on the assessment result, implementing the selected state (5) on a quantum computer (3), and estimating the ground state energy (10) of the quantum chemical system on the quantum computer based on the implemented state (5). Further, the invention relates various methods for preparing a quantum state (4,5) for quantum energy estimation on a quantum chemical system and to a system (1).

## Description

The invention relates to a method for estimating a ground state energy of a quantum chemical system and a system. Further, the invention relates to methods for preparing a quantum state for quantum energy estimation on a quantum chemical system.

There is a lot of interest in obtaining good estimates of the ground state energies of electronic systems, due to the relevance to practical applications, such as design of new chemical reactions, catalysts, materials, etc.

Preparing an initial state with high enough quality is an indispensable ingredient for the current methods of quantum energy estimation. Conventionally the energy estimation is considered for the ground states of many body systems and the quality of the initial state is assessed by how large of an overlap it has with the ground state of the system. The methods, ranging from quantum phase estimation and its variants [1, 2] to more recent developments, all require a lower bound on the overlap with the target state whose energy is being estimated.

In all these schemes the lower bound on the overlap also appears in the cost directly, usually through the factor 1/overlap² (or 1/overlap when a method such as amplitude amplification is used) which determines the number of repetitions that is required for obtaining the energy of the target state.

This signifies the importance of preparing a high-quality initial state for the efficiency and relevance of the whole quantum energy estimation procedure.

When it comes to how state preparation should be done in practice, many different methods may be considered, methods ranging from quantum heuristic algorithms such as adiabatic state preparation, variational methods, quantum imaginary time evolution, to quantum preparation of a classically computed ansatz, etc. The latter by itself can take many different forms based on the classical ansatz considered for implementation, e.g., Hartree-Fock, (unitary) coupled cluster, sums of Slater determinants [3], matrix product states [4, 5], etc. However, even though there are a variety of possibilities of different classical ansatzes, most prior art just focuses on implementing Hartree-Fock states which are known to be poor representations for strongly correlated many-body systems, the main candidates for useful application of quantum energy estimation routines. Thus there's a demand for improvement.

The invention is based on the technical problem of improving a method and a system for estimating a ground state energy of a quantum chemical system.

According to the invention, the technical problem is solved by a method having the features of claim 1 and a system having the features of claim 15. Further, the technical problem is solved by methods according to claims 8, 11, and 12. Advantageous embodiments of the invention emerge from the dependent claims.

In particular, a method is provided for estimating a ground state energy of a quantum chemical system, the method comprising:
- classically computing states close to the ground state of the quantum chemical system,
- assessing the quality of the classically computed states based on the energy distribution of each state,
- selecting a state based on the assessment result,
- implementing the selected state on a quantum computer, and
- estimating the ground state energy of the quantum chemical system on the quantum computer based on the implemented state.

Further, in particular a system is provided, comprising:
- a classical computer, and
- a quantum computer,
wherein the classical computer is configured to perform the classical computations of a method according to any one of the embodiments described in this disclosure, and wherein the quantum computer is configured to perform the quantum computations of the method.

One of the main ideas of the invention is to compute, using classical (non-quantum) methods on a classical computer, candidate states which are close to the ground state of the quantum chemical system for which the ground state energy is to be estimated. After calculating a variety of candidates for later implementation on the quantum computer, the candidate most suitable is selected. To this end, the quality of each classically computed state is assessed based on the energy distribution of the respective state. The best candidate is selected based on the assessment result. In other words, with the energy-distribution-based quality assessment technique, it is possible to decide between different possible classically found candidates and choose the one with highest quality. The selected state is implemented on the quantum computer and the ground state energy of the quantum chemical system is estimated on the quantum computer based on the implemented state. In other words, the selected state is used as the initial state that is propagated through the circuit of the quantum computer in order to find the ground state energy.

The invention is, in particular, based on the finding that the conventional criterion, i.e., evaluating the overlap with the ground state, is a hard task and is in fact most of the time in conflict with the whole purpose of performing quantum energy estimation for the given problem, since it needs a great deal of knowledge about the ground state of the system in question. In particular, it is found that it is preferable to assess the quality of the candidate states based on the (full) energy distribution. This is partly because unlike the ground state overlap there are ways to obtain approximated energy distributions both classically and quantumly. Furthermore, enough information for assessing the quality of the state can be obtained from the energy distribution. In particular, the accumulated weight of the energy distribution for low energies, also referred to as the support on the low-energy subspace, is an important quantity to consider as this is proportional to the probability of obtaining energy estimates as low as those energies in a quantum routine. In particular, the candidate state is chosen that has the largest weights for smaller energies (see examples below).

To calculate the states close to the ground state of the quantum chemical system, a Hamiltonian describing the system is defined and a traditional quantum chemistry method is executed on a classical computer to obtain an approximate solution for the ground state (i.e., the system is solved classically on a classical computer). In particular, the classical computation is performed to obtain an initial state that potentially has large support on the low-energy subspace of the Hamiltonian (e.g., the ground state). Different classical methods, among others, may be used to perform the classical calculation, for example: Hartree-Fock, Configuration Interaction with singles and doubles (CISD), Coupled Cluster (CC) with single or double excitations (CCSD), complete active space configuration interaction (CASCI), multireference perturbation theory (MRPT), selective configuration interaction methods, such as semi-stochastic heat bath configuration interaction (SHCI), and/or tensor network ansatz methods, in particular density matrix renormalization group (DMRG).

Finally, the ground state energy estimated by the method described in this disclosure is provided. In particular, a signal and/or data is provided which describes the estimated ground state energy.

### Energy distribution

In the following, the procedure for deriving and estimating the energy distribution is described.

The energy distribution of a candidate initial state |*ψ*〉 with respect to the Hamiltonian H may be defined in the following way: $A \left(ω\right) = {\sum }_{E} {\left|〈E | ψ〉\right|}^{2} {f}_{η} \left(E-ω\right)$
where *E,* |*E*〉 are eigenvalues and eigenstates of *H* and *f_{η}* is some kernel, e.g., Gaussian, Lorentzian, etc., with width *η* centered at the position of each of the eigenvalues. *η* = 0 corresponds to a discrete distribution, the actual energy distribution of the state, while with *η* ≠ 0 each energy value is broadened and the result is a continuous distribution. In practice, it will be hard to access the true *η* = 0 distribution and focus should thus be on broadened versions.

A simple criterion based on the energy distribution can be formulated for the quality of states for performing energy estimation; supposing a number of candidate initial states are given with similar energies, i.e., with similar expectation values of *H*, the quality can be compared by focusing on their distribution and in particular on their left-side (i.e., low-energy-side) tails. Roughly, whichever state that has more weight extended to lower energies is a better candidate as it provides higher probability for obtaining a low energy estimate in the energy estimation process.

In particular, assessing the quality comprises, for each potential classically computed candidate state: calculating a distribution of QPE outcomes of N measurements based on the energy distribution of the potential candidate state, calculating a cumulative distribution function of the minimum energy of each QPE outcome with respect to a reference energy *E*₀, differentiating the cumulative distribution function with respect to the reference energy *E*₀, and determining the properties of the lowest possible outcome based on the differentiated cumulative distribution function. In particular, the lowest possible outcomes of the candidate states are compared with each other, in particular with the goal of choosing the smallest one.

In particular, selecting a state may comprise using the mean value of the differentiated distribution and choosing the candidate state with the smallest mean value.

### Using energy distribution for estimating lowest outcomes

In the following, this is described in more detail. Assuming one has access to the (approximate) energy distribution of the candidate state of interest, one can calculate the distribution of QPE outcomes. It can be calculated as follows for k digits and an integer outcome *xₒ*: ${{A}^{˜}}_{QPE} \left({x}_{o}\right) = \int dE A \left(E\right) \frac{1}{{2}^{2 k}} \left(\frac{{sin}^{2} \left(π{2}^{k}E\right)}{{sin}^{2} \left(π\left[E-{x}_{o}/{2}^{k}\right]\right)}\right)$

It is noted that this is a discrete distribution function. In the following, the distribution of potential outcomes of QPE is studied, and in particular the lowest possible measurements that are expected to be obtained. Since high-precision QPE is performed and for simplicity of presentation, an approximation is used and the continuous limit of the outcome distribution with *Eₒ* = 2*^{-k}xₒ* is taken. Then defining *A*_{QPE}(*Eₒ*) = 2*^{-k}Ã_{QPE}*(*xₒ*), it is noted that in the limit of *k* → ∞, *A*_{QPE} approaches *A* and thus *A*(*E*) is used in the following discussion. It is straightforward to undo this and work with the actual discrete QPE outcome distribution when needed.

First, one would like to understand the statistics of the best energy achievable through a number of QPE measurements *N;* supposing the QPE outcomes are *E*₁, *..., E_{N}*, one would like to focus on the distribution of ${E}_{min}^{\left(N\right)} = min \left({E}_{1},…,{E}_{N}\right)$. It is straightforward to calculate the cumulative distribution function of this variable at energy *E*₀ as: ${C}_{{min}_{N}} \left({E}_{0}\right) = 1 - {\left(1-{p}_{<}\left({E}_{0}\right)\right)}^{N}$

This is the probability that at least one outcome from N rounds of QPE lies below *E*₀: here $p < \left({E}_{0}\right) = {\int }_{- ∞}^{{E}_{0}} dE A \left(E\right)$ is the probability of a single outcome lying below *E*₀. In this way, one can see that *C*_{min*_{N}*} is also the probability that the smallest outcome from the list *E*₁, *...*, *E_{N}* is below *E*₀: either there is only one, and it by definition the smallest, or there are several and then one of them is the smallest - but they are all definitely below *E*₀. Upon differentiating the cumulative distribution function with respect to *E*₀, one obtains the probability distribution function of min(*E*₁, ...,*E_{N}*), which reads: ${A}_{min}^{\left(N\right)} \left({E}_{0}\right) = NA \left({E}_{0}\right) {\left(1-{p}_{<}\left({E}_{0}\right)\right)}^{N - 1}$

With this probability distribution function, one can determine the properties of the lowest possible outcome from N QPE measurements, and in doing so, compare different candidate states associated with these distributions and choose the best one. In particular, one simple measure of state quality through this approach may be the mean value of this distribution: $\int {dEA}_{min}^{\left(N\right)} \left(E\right) E$. A state whose associated mean value is smaller is judged to be better suited for running QPE.

Another way of understanding the statistics of the lowest outcome from N rounds of QPE measurements is as follows. Consider again the probability of the smallest of N outcomes lying below *E*₀ shown in Eq. (3). Suppose one would like to find the threshold *E*₀, beyond which this probability falls below 1 - *δ*, with 0 < *δ* < 0.5, and take this threshold as a measure of how small of an outcome is probable for a given energy distribution. This threshold can be found as the following; we find the *E*₀ such that the following equations hold: $\begin{matrix}{C}_{{min}_{N}} \left({E}_{0}\right) = 1 - δ \\ {\left(1-{p}_{<}\left({E}_{0}\right)\right)}^{N} = δ \\ N = log δ / log \left[1-{p}_{<}\left({E}_{0}\right)\right]\end{matrix}$

For large N, one expects *p*_{<}(*E*₀) to be small and thus Taylor expansion of the logarithm results in $N = O \left(log\left(\frac{1}{δ}\right)\frac{1}{p < \left({E}_{0}\right)}\right)$. Solving the last row of Eq. (4) for *E*₀ gives the desired threshold beyond which the probability of obtaining an outcome becomes smaller than 1 - *δ*.

Now, the quality of two energy distributions *A*₁ and *A*₂ may be compared; two methods are discussed for this. First, to this end, the probability of obtaining a lower outcome from distribution *A*₂ is considered, which can be written as: ${I}_{1 ; 2} = {\int }_{- ∞}^{∞} {dE}_{1} {A}_{1 , min}^{\left(N\right)} \left({E}_{1}\right) {\int }_{- ∞}^{{E}_{1}} {dE}_{2} {A}_{2 , min}^{\left(N\right)} \left({E}_{2}\right)$

Depending on whether *I*_{1;2} is larger or smaller than $\frac{1}{2}$, *A*₂ or *A*₁ is more capable of producing lower energies with N QPE measurements.

Another simple criterion can be given by the threshold obtained from Eq. (4). With a choice of *δ*, whichever probability distribution gives a smaller threshold can be chosen as the higher quality one.

In one embodiment, the method further comprises approximating the energy distributions of at least a subset of the classically computed states through an Edgeworth series expansion using the Hamiltonian moments of the state and/or by the resolvent (matrix product state-based) method. These are classical methods which are implemented on the classical computer.

### Edgeworth series

For the Edgeworth series, the moments of energy (expectation values of powers of H) are used and series expansions are obtained for the energy distribution. The Edgeworth series and the Gram-Charlier series are considered for illustration. Both approximate a distribution as a Gaussian multiplied by different orders of the Hermite polynomials. The coefficients of the terms in the series, can be written in terms of the moments of the distribution, or in other words the expectation values of powers of the Hamiltonian 〈*ψ*|*Hⁿ*|*ψ*〉. The lowest order approximation in this scheme is a Gaussian distribution with a variance proportional to 〈*ψ*|*H*²|*ψ*〉. It is noted that this method works best for distributions that are close to Gaussian. In the following, these series expansion techniques are discussed.

The Edgeworth and Gram-Charlier series have identical terms, the only difference being that the terms in an Edgeworth series are arranged in a way that the series constitutes a true asymptotic series [12]. First, details on the Gram-Charlier series are given; the expansion can be written as: ${P}_{GS} \left(x\right) = \frac{{e}^{- {x}^{2} / 2}}{\sqrt{2 π}} \left[1+{\sum }_{n = 3}^{∞} {\left(-1\right)}^{n} {c}_{n} {He}_{n} \left(x\right)\right]$ where *Heₙ*(*x*) is the Hermite polynomial in the probabilist's notation defined as: ${He}_{n} \left(x\right) = {\left(-1\right)}^{n} {e}^{\frac{{x}^{2}}{2}} \frac{{d}^{n}}{{dx}^{n}} {e}^{- \frac{{x}^{2}}{2}}$ $\int dx \frac{{e}^{- {x}^{2} / 2}}{\sqrt{2 π}} {He}_{n} \left(x\right) {He}_{m} \left(x\right) = {δ}_{nm} n !$

The expansion in the form in Eq. (5) is used for a distribution function with zero mean and unit variance; it is noted that any distribution satisfies these two criteria upon translating and rescaling.

Using the above orthonormality relations, one is able to determine the coefficients of the Gram-Charlier expansion for a distribution function p(x) order by order as follows: ${c}_{n} = \frac{{\left(-1\right)}^{n}}{n !} \int dx p \left(x\right) {He}_{n} \left(x\right)$

It is clear that the coefficients can be written in terms of the moments of the distribution *µₙ* = *∫ dx p*(*x*) *xⁿ*. A list of the coefficients is given in Table 1.

**Table 1: Coefficients cₙ defined in the equation mentioned above.**

| order | Gram-Charlier coefficient |
|---|---|
| 3 | $- \frac{1}{3 !} {μ}_{3}$ |
| 4 | $\frac{1}{4 !} \left[{μ}_{4}-3\right]$ |
| 5 | $\frac{1}{5 !} \left[-{μ}_{5}+10{μ}_{3}\right]$ |
| 6 | $\frac{1}{6 !} \left[{μ}_{6}-15{μ}_{4}+30\right]$ |
| 7 | $\frac{1}{7 !} \left[-{μ}_{7}-21{μ}_{5}-105{μ}_{3}\right]$ |
| 8 | $\frac{1}{8 !} \left[-{μ}_{8}-28{μ}_{6}-210{μ}_{4}-315\right]$ |

On the other hand, the Edgeworth series as mentioned above is an asymptotic series that is obtained by grouping the same terms in a Gram-Charlier series in a particular way: ${P}_{E} \left(x\right) = \frac{{e}^{- {x}^{2} / 2}}{\sqrt{2 π}} \left[\begin{matrix}1 + \frac{{κ}_{3}}{6} {He}_{3} \left(x\right) + \left(\frac{{κ}_{4}}{24}{He}_{4}\left(x\right)+\frac{{κ}_{3}^{2}}{72}{He}_{6}\left(x\right)\right) \\ + \left(\frac{{κ}_{5}}{120}{He}_{5}\left(x\right)+\frac{{κ}_{4} {κ}_{3}}{144}{He}_{7}\left(x\right)+\frac{{κ}_{3}^{3}}{1296}{He}_{9}\left(x\right)\right) + ⋯\end{matrix}\right]$ where *κ*ₙ is the *n*th cumulant of the distribution: ${κ}_{3} = {μ}_{3}$ ${κ}_{4} = {μ}_{4} - 3$ ${κ}_{5} = {μ}_{5} - 10 {μ}_{3}$

For the general prescription for obtaining the terms and explicit forms for more terms, see Appendix A. In practice, in some cases, the above series expansions exhibit irregular behaviors like having negative values, artificial rapid oscillations, etc. This happens mostly in cases where the energy distribution is very far from a Gaussian. Large gaps in the spectrum can also result in such behavior.

Generally, the series will converge if the approximated distribution functions falls faster than exp(-*x*²/4) for large x [12]. Even if the above series do not converge, it is still possible to truncate them, particularly the Edgeworth series as it is an asymptotic series, and still obtain some useful information. The convergence condition is satisfied for a bounded energy spectrum. However, the discreteness of the true energy distribution can be problematic for the approximation. Here, focus is set on computation of moments of energy through the matrix product state (MPS) language. This should not impose a large computational overhead as acting with the Hamiltonian matrix product operator on the solution MPS even multiple times is not a prohibitive task. Particularly, in practice it can be seen that it is simple to go moments of order ~10 without a need to increase the state's bond dimension substantially. The lowest order approximation in the series corresponds to a Gaussian fitting to the distribution and the only information that is required for it is the variance of energy in the initial state 〈*ψ*|*H*²|*ψ*〉.

### Resolvent method

The other classical approach is the resolvent method. This is a classical computational method in which the goal is to find the Green's function defined as below: $G \left(ω, η\right) = \frac{1}{π} 〈 ψ | \frac{1}{H - ω + iη} | ψ 〉$ where *η* is a broadening factor. From this Green's function, one can get the energy distribution function for energy *ω* as the following: $A \left(ω, η\right) = - Im G \left(ω, η\right)$

It is noted that since $Im \frac{1}{E - ω + iη} = \frac{- η}{{\left(E-ω\right)}^{2} + {η}^{2}}$ this method amounts to using a Lorentzian kernel with broadening *η* in Eq. (1).

The above Green's function is solved for through a DMRG-like variational method that was introduced in Ref. [13] and then improved in Ref. [14]. The former method is called dynamical DMRG (DDMRG) and the improved version is called DDMRG++ in Ref. [14]. It is an MPS method and performs DMRG-like sweeps to evaluate the above Green's function. This means that the state that is to be implemented on the quantum computer needs to be transformed into MPS form, which is possible as discussed earlier.

The method works as follows; the state |*ϕ*〉 is defined that satisfies: $| φ 〉 = \frac{1}{π} \frac{1}{\left(H-ω+iη\right)} | ψ 〉 ⇒ π \left(H-ω+iη\right) | φ 〉 = | ψ 〉$

The above Green's function can be written as the overlap *G*(*ω*,*η*) = 〈*ψ*|*ϕ*〉*.* Now, defining |*Y*〉 as [13]: $\left[{\left(H-ω\right)}^{2}+{η}^{2}\right] | Y 〉 = - \frac{η}{π} | ψ 〉$

It is easy to see that Im *G =* 〈*ψ*|*Y*〉. Finding the overlap of the above equation with |*Y*〉, a DMRG-like algorithm is used to minimize the resulting functional [13, 14]: $〈 Y | \left[{\left(H-ω\right)}^{2}+{η}^{2}\right] | Y 〉 + \frac{η}{π} 〈 Y | ψ 〉$

For example, the package block2 [6] may be used for implementation of DDMRG++. Even though the method is implemented in the MPS language, it can be thought of as general because as discussed in Appendix B, there are methods for transforming other forms of states into MPS. It is also mentioned that the same Green's function can be calculated using time evolution, namely calculating the overlap 〈*ψ*|*e^{iHt}*|*ψ*〉 and then Fourier transforming (with the addition of a damping function *e*^{-*ηt*}). Similar MPS-based methods can also be used to this end [14].

In one embodiment, the method further comprises approximating the energy distributions of at least a subset of the classically computed states through coarse quantum phase estimation (QPE). It is noted that the classically computed states must be implemented on a quantum computer in order to perform coarse QPE. With a quantum computer capable of performing precision energy estimation, it is expected to also be able to perform lower precision QPE. Performing lower precision (coarse) QPE can give an approximate energy distribution function. In the following, a method that approximates an energy distribution in the form of Eq. (1) with *f_{η}* given by the QPE kernel is described: ${K}_{QPE} \left(ω\right) = \frac{1}{{2}^{2 k}} \frac{{sin}^{2} \left(π{2}^{k}ω\right)}{{sin}^{2} \left(πω\right)}$ for some integer k that is the number of digits in the coarse QPE measurement. One possible way to obtain an energy distribution is through performing QPE multiple times, obtaining the statistics of the outcomes, and then trying to reconstruct the energy distribution from that. However, the outcomes are discrete and one needs to consider further manipulations to obtain an unbiased distribution function. It is noted that the energy levels themselves are also discrete. Here, in particular a method is proposed so that, as mentioned above, each energy level is broadened by the QPE kernel.

For this, performing QPE multiple times is considered, but here for each QPE round, in particular a random constant c is added to the Hamiltonian under which the QPE time evolution is performed. The constant c is chosen to lie in the interval [0, 2^{-*k*}) (since it is a coarse QPE measurement, these constants are not negligible). Then, after the measurement is performed and an integer *xₒ* is observed in the phase register of QPE, the measured energy is taken to have the value 2⁻*^{k}xₒ* - *c*. In this way, all real values are possible to be sampled and from performing the measurements a number of times, one is able to approximate the energy distribution using smoothing methods such as kernel density estimation [15].

In the following, a quick overview of the kernel density approximation method is given. Supposing access to a finite number of samples drawn from a distribution function is available, the goal is to approximate the distribution function. To this end, a broadening kernel is placed at the position of each of the outcomes and a normalized sum approximates the underlying distribution: $\hat{p} \left(x\right) = \frac{1}{nh} {\sum }_{i = 1}^{n} K \left(\frac{x - {X}_{i}}{h}\right)$ where K is a kernel (e.g., Gaussian, Lorentzian, etc.) with mean of 0 and variance of 1; *Xᵢ,i* = 1,..., *n* are the outcomes of sampling and *h* is the broadening factor.

The analysis of error in reconstructing the above QPE kernel energy distribution with kernel density estimation follows a standard approach [15]. First, the error is quantified by the mean integrated square error (MISE): $MISE = E \left(\int dx {\left(\hat{p}\left(x\right)-p\left(x\right)\right)}^{2}\right)$ where p(x) is the approximated distribution for the underlying distribution p(x). When a sample of size *n* is used, it is well known that with an appropriate choice of *h*, i.e., *h*ₒₚₜ∼1/*n*^{1/5}, the error shows the behavior MISE∼1/*n*^{4/5}.

In one embodiment, implementing the selected state comprises preparing and using a compressed representation of the sum of Slater determinants. This way a representation can be found that has a dimension smaller than that of the Hilbert space. In particular, using the compressed representation of the sum of D Slater determinants requires a Toffoli cost of *O*(*D* log *D*). This embodiment is particularly suited for implementing a state in a large Hilbert space, which is a sum of a number of product states much smaller than the overall Hilbert space dimension.

In particular, a simpler way to read the system register is used by adding a smaller auxiliary register and transferring the information into the smaller auxiliary register, which is then more easily read.

In the following, this embodiment is described in more detail. In the description provided a second quantized language is used, but the implementation method is general and in particular can also be used for a first quantized implementation as well.

The normalized state ${\sum }_{i = 1}^{D} {α}_{i} | {a}_{1 , i} {a}_{2 , i} … {a}_{2 N , i} 〉$ needs to be implemented where *αᵢ* are the given amplitudes and |*α*_{1,}*ᵢα*_{2*,*i} ... *α*_{2*N,i*}〉 are orbital occupation bitstrings of length 2N qubits and denote occupation of orbitals with spin ↑ or ↓. It is noted that N is the total number of orbitals. Interest is set in cases where the number of terms in the sum of Slater determinants D is much smaller than the Hilbert space dimension 2^{2*N*}. Therefore, the problem is to implement a summation of relatively few basis states picked from a very large Hilbert space.

Roughly, the implementation may work as follows. The aim is to implement the state ${\sum }_{i = 1}^{D} {α}_{i} | {v}_{i} 〉$ where |*vᵢ*〉 represents the orbital occupation bitstrings of length 2N mentioned above. To this end, the state ${\sum }_{i = 1}^{D} {α}_{i} | i 〉$ is implemented using the quantum read-only memory (QROM) method in Ref. [8]. This will cost ∼2^{*d*+1} Toffoli gates for $d = ⌈ {log}_{2} \left(D\right) ⌉$, where the enumeration register |i〉 has d qubits. Then a (Select or SelSwapDirty) QROM oracle *O* is used, of Toffoli cost 2*^{d}*, that reads |*i*〉 and outputs the corresponding orbital occupation bitstrings into the system register of size 2N: $O | i 〉 {| 0 〉}^{⊗ 2 N} = | i 〉 | {v}_{i} 〉$ which results in the following state: ${\sum }_{i = 1}^{D} {α}_{i} | i 〉 | {v}_{i} 〉$

Now, the enumeration register denoted by |*i*〉 in the above state needs to be uncomputed and disentangled, i.e., an operator 7 needs to be built such that $V | i 〉 | {v}_{i} 〉 = {| 0 〉}^{⊗ d} | {v}_{i} 〉$

For this, a naive approach is to use QROM one more time on the system register, i.e., |*vᵢ*〉 orbital occupation bitstrings; this will induce a cost of 2^{2*N*} which is exponentially large in the system size and can be prohibitive; so, an alternative method that leverages the fact that the number of terms D is much smaller than this Hilbert space dimension is used.

In particular, the following solution is used. The general idea is to efficiently construct a more compressed representation of all the Slater determinants (represented by $⌈ {log}_{2} \left(D\right) ⌉$ qubits) that can be used in the algorithm instead of the full Slater determinants of size 2N qubits. This will help reduce gate costs, as well as qubit cost, when multi-controlled CNOT gates are used.

To this end, the following matrix is considered ${\left({v}_{i}…{v}_{D}\right)}_{n \times D}$ with r its rank, and where *vᵢ* are put as column vectors. By elementary linear algebra, a new matrix with rank r can be constructed by picking only r rows of the above matrix; in other words, there are substrings *ṽᵢ* of *vᵢ* of length r such that ${\left({{v}^{˜}}_{1}…{{v}^{˜}}_{D}\right)}_{r \times D}$ has rank r. Notice *r* ≤ min(*n*,*D*). The substrings *ṽᵢ* in the above equation are bits at specific locations 1 ≤ *l*₁ <... < *lᵣ* ≤ *n* of *vᵢ*; the indices *l*₁,... , *lᵣ* are identical for all *vᵢ*. Assuming the following lemma, it can be shown in the following corollary how the operator 7 can be built.

Lemma: There exists a classical algorithm with polynomial complexity in terms of (*r*,*D*), that outputs 2d - 1 bitstrings *uⱼ* of length r, such that the bitstrings *bᵢ =* (*u*₁ · *ṽᵢ,* ..., *u*_{2*d*-1} · *ṽᵢ*) presented as column vectors in ${\left(\begin{matrix}{u}_{1} \\ ⋮ \\ {u}_{2 d - 1}\end{matrix}\right)}_{\left(2d-1\right) \times r} {\left(\begin{matrix}{{v}^{˜}}_{1} & … & {{v}^{˜}}_{D}\end{matrix}\right)}_{r \times D} = {\left(\begin{matrix}{b}_{1} & … & {b}_{D}\end{matrix}\right)}_{\left(2d-1\right) \times D}$ are mutually distinct. The above dot products and matrix multiplication are performed modulo 2.

Corollary: Using the *uᵢ* in the lemma, one can build the operator 7 with a Toffoli cost of (2*d* - 1) · *D* and additional qubit cost of 4d - 3.

Proof: Recall that the state is ${\sum }_{i = 1}^{D} {α}_{i} | i 〉 | {v}_{i} 〉$. Let the system register be denoted simply by *v* with *v*^{(*k*)} being the *k*-th qubit. Let | ·〉*_{b}* be an ancilla qubit initialized at 10). The aim is to output (*u*₁)₁ · (*ṽ*ᵢ)₁ inside the *b* register. Since (*u*₁)₁ is known, if it is zero, that means leaving the *b* register untouched, and if it is one, it means a CNOT with control on *v*^{(*l*₁)} and target *b*. One gets ${\sum }_{i = 1}^{D} {α}_{i} | i 〉 {| {v}_{i} 〉}_{v} {| {\left({u}_{1}\right)}_{1} ⋅ {\left({{v}^{˜}}_{i}\right)}_{1} 〉}_{b}$

Then with another identity map or CNOT with control on *v*^{(*l*₂)} and target *b*, the state becomes ${\sum }_{i = 1}^{D} {α}_{i} | i 〉 {| {v}_{i} 〉}_{v} {| {\left({u}_{1}\right)}_{1} ⋅ {\left({{v}^{˜}}_{i}\right)}_{1} + {\left({u}_{1}\right)}_{2} ⋅ {\left({{v}^{˜}}_{i}\right)}_{2} 〉}_{b}$

It is easy to see that repeating this process r times, delivers the state ${\sum }_{i = 1}^{D} {α}_{i} | i 〉 {| {v}_{i} 〉}_{v} {| {u}_{1} ⋅ {{v}^{˜}}_{i} 〉}_{b} = {\sum }_{i = 1}^{D} {α}_{i} | i 〉 {| {v}_{i} 〉}_{v} {| {\left({b}_{i}\right)}_{1} 〉}_{b}$

If the register *b* is expanded to include 2d - 1 qubits, one could do the above for all of *u*₂, ..., *u*_{2*d*-1} to get the state: ${\sum }_{i = 1}^{D} {α}_{i} | i 〉 {| {v}_{i} 〉}_{v} {| {\left({b}_{i}\right)}_{1} … {\left({b}_{i}\right)}_{2 d - 1} 〉}_{b}$

It is noted that this is all done without any Toffoli so far. Rewriting, one gets ${\sum }_{i = 1}^{D} {α}_{i} | i 〉 {| {v}_{i} 〉}_{v} {| {b}_{i} 〉}_{b}$

Now *bᵢ* in register *b* acts as a unique small-sized flag for *vᵢ*. For each *bᵢ*, by using a multi-controlled-NOT of order 2*d* - 1, one can flip the register *i* to all zero. This uses (2*d* - 1) · *D* Toffolis and 2*d* - 2 additional qubits.

Of course, one also has to disentangle the register *b*, which is done by its uncomputation. The total Toffoli cost therefore stays at (2*d* - 1) · *D* with an additional qubit cost of (2*d* - 1) + (2*d* - 2) = 4*d* - 3, where the term (2*d* - 1) comes from the register *b* and the term (2*d* - 2) comes from the multi-controlled-CNOTs.

In one embodiment, implementing the selected state comprises preparing and using a matrix product state (MPS) form. In particular, a state in the MPS form is implemented using an algorithm in which the matrices comprising the MPS are used directly to synthesize unitary operations required to construct the state one at a time. Direct implementation of an MPS can also impose less cost over implementing a sum of Slater determinant state in some cases. These cases are, for example, strongly correlated / multireference systems (e.g., transition-metal dimers like Cr₂ / Fe₂, or clusters such as the iron-sulfur clusters [Fe₂S₂] and [Fe₄S₄] and FeMoco / nitrogenase, Hubbard models, etc.). The reason is that in such systems, it will take a large number of Slater determinants (-10,000 or more) to encode enough of the wavefunction to get a good quality state, and the cost of Slater implementation scales with the number of determinants. In contrast, MPS can generically represent ground states of most chemical Hamiltonians of interest accurately enough with polynomial (in system size) number of parameters and cost, but translating the same MPS into a sum of Slater determinants form can result in exponentially (in system size) many parameters required.

In the following, the implementation using MPS is described in more detail. Mainly the method introduced in Ref. [4] is considered for this task and to do a Toffoli gate cost estimation. It is noted that there are many variations of this method considered in Refs. [9-11] as well as newer versions requiring lower depth circuits [5] for short range correlated MPS.

First, a quick review of the method in Ref. [4] is given. Starting with the MPS form $\begin{matrix}| ψ 〉 = {\sum }_{{n}_{1} , … {n}_{N}} {\sum }_{{α}_{1} , … , {α}_{N - 1}} {A}_{1 ; {α}_{1}}^{{n}_{1}} {A}_{2 ; {α}_{1} {α}_{2}}^{{n}_{2}} … {A}_{N ; {α}_{N - 1}}^{{n}_{N}} | {n}_{1} , {n}_{2} , … , {n}_{N} 〉 \\ {n}_{i} ∈ \left\{vac,↑,↓,↑↓\right\}\end{matrix}$ the standard graphical notation for manipulation of the MPS is used. Tensors are denoted as

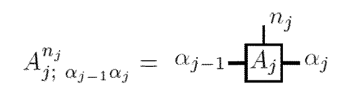

Where the physical index *nⱼ* runs over d values, and where d is the local Hilbert space dimension and the auxiliary indices *α*_{*j*-1}, *αⱼ* run over *χ* values, where *χ* is the bond dimension. It is possible for different *αⱼ*'s to run over different numbers, which are denoted collectively as *χ* above. The whole MPS is turned into a left canonical form:

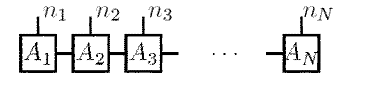

For all j > 1, one has ${\sum }_{{α}_{j} , {n}_{j}} {A}_{j ; {α}_{j - 1} {α}_{j}}^{{n}_{j}} {\left({A}_{j ; {α}_{j - 1}^{′} {α}_{j}}^{{n}_{j}}\right)}^{∗} = {δ}_{{α}_{j - 1} α {′}_{j - 1}}$ or diagrammatically:

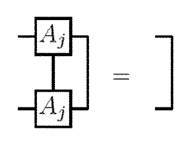

In the above equations, for the last tensor *A_{N}* the summation over the right auxiliary index can simply be dropped.

The method essentially works by first observing that the above tensors of the MPS, owing to the left canonical form, can be directly used to define unitaries *G* that are used in a quantum circuit for implementing the MPS: $G {\left[j\right]}_{{α}_{j} {n}_{j} , {α}_{j - 1} 0} = {A}_{j ; {α}_{j - 1} {α}_{j}}$

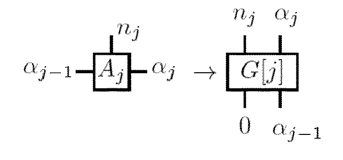

It is noted that each unitary acts on a *d*-level qudit or log₂(*d*) physical qubits and log₂ *χ* ancillae. For example, for fermionic systems, *d* = 4 and two qubits are required for each orbital of the system (each qubit can be assigned to either spin ↑ or ↓). The incoming physical index for the unitary is set to be equal to 0 and thus the above relation does not specify all the elements of *G[j].* This is fine as long as the rest of the elements are chosen so that *G[j]* remains unitary. It is noted that Eq. (3) ensures that the specified elements of *G[j]* satisfy the required unitarity relations: ${\sum }_{{α}_{j} {n}_{j}} G {\left[j\right]}_{{α}_{j} {n}_{j} , {α}_{j - 1} 0} {\left(G{\left[j\right]}_{{α}_{j} {n}_{j} , α {′}_{j - 1} 0}\right)}^{∗} = {δ}_{{α}_{j - 1} α {′}_{j - 1}}$

A quantum circuit that implements the desired MPS and works with these unitaries is shown in the schematic below. It is noted that an auxiliary register of a size denoted as $⌈ {log}_{2} χ ⌉$ is required to reconstruct the MPS and that it is schematically moved around to act with unitaries on this register and different qubits in the system. As the bond dimension can change as the circuit traverses through the system, one can start with a number of ancillae equal to $⌈ {log}_{2} {χ}_{max} ⌉$ and use more or fewer of the available ancillae as the bond dimension dictates. Also, it is noted that for the first and the last unitaries the input and output auxiliary registers also have value 0.

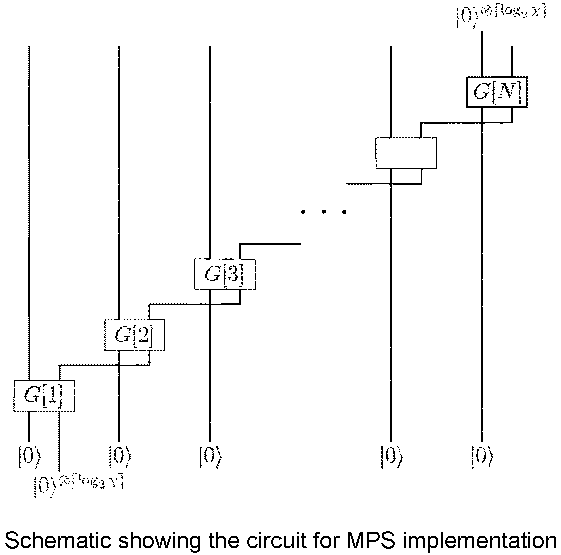

For the above circuit one needs to synthesize the unitaries *G*[*j*] for which the unitary synthesis method of Ref. [8] may be used.

In one embodiment, the method further comprises quantum refining of the implemented state on the quantum computer by filtering out higher energy contributions after state implementation. This way the cost of ground state energy estimation may be further decreased. In particular, this decreases the cost by decreasing the number of times the most accurate quantum phase estimation (QPE) has to be performed on the quantum computer in order to get the final result.

Particularly, the aim is to filter out some of the remaining high-energy weight in the energy distribution quantumly. If the quality of the state is improved, meaning the low-energy weight is increased, the cost of the whole procedure decreases as the number of times the most precise energy estimation algorithm needs to be repeated is lowered. This is by itself particularly beneficial if a cheap quantum refining procedure can be used.

Furthermore, the leakage problem of QPE can also be addressed using quantum refining which means that each single round of the most precise QPE measurement becomes less costly apart from decreasing the total number of repetitions. The leakage problem of QPE is a known problem that is usually considered in the literature as changing the cost scaling of QPE to a $1 / {p}_{0}^{2}$ behavior instead of 1/*p*₀, where *p*₀ is the square of the overlap of the initial state with the ground state (see I.A of [22] and Appendix A of [23]). This scaling, as is argued in the literature, happens when long tails of QPE kernels placed at higher energies contribute outcomes at low energies, potentially even below the ground state value.

In principle, the leakage probability can be suppressed through the quantum refining discussed above. As the probability of leakage from low energy states themselves can be suppressed by neglecting an 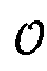 (1) number of digits from QPE, the leakage mostly comes from the tail of far-away weight, i.e., from the algebraically decaying QPE kernel. As a result, if the far-away weight responsible for leakage is suppressed through quantum refining, the leakage probability is also suppressed with it. Identifying and filtering out the most relevant weight thus becomes a matter to be studied on a case-by-case basis. However, the costs for such refining can be a lot smaller when compared with the ultimate precision QPE of interest. As an example, considering an energy distribution having a multimodal structure, one can identify the peaks, with 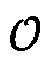 (1) probability, that are responsible for leakage; then one can perform a quantum refining, e.g., in the form of coarse QPE, in a way such that those peaks are well suppressed after appropriate postselection. For example, one can tune the QPE (number of digits, constant, etc.) in a way such that the most important peaks are close to QPE outcomes that are discarded. Thus, when postselecting over desired outcomes, these peaks will be suppressed. After this filtering, the probability of leakage can be assessed again and, in case leakage is still probable, further quantum refining can be used, e.g., with higher digits for the coarse QPE. Note that this means the quantum refining step of the state preparation method is not only capable of reducing the cost by lowering the number of required repetitions of the most precise QPE, but also by decreasing the cost of each single round by mitigating the leakage problem.

To this end, a variety of quantum techniques may be employed. In one embodiment, quantum refining comprises using at least one of: Hamiltonian polynomial methods, coarse quantum phase estimation, quantum imaginary time evolution, and in particular quantum eigenvalue transformation of unitary matrices with real polynomials (QETU).

Two of these methods are briefly described in the following: coarse QPE and quantum eigenvalue transformation of unitary matrices with real polynomials (QETU), where the latter is chosen as a representative of the polynomial-based algorithms.

### Coarse QPE with postselection

In this method, coarse QPE is performed, i.e., a QPE operation with fewer digits than in the ultimate-precision QPE measurement. In each coarse QPE measurement, if the outcome corresponds to high energies, the state is discarded and the quantum implementation is restarted. It is the smaller energy outcomes of QPE that one aims to postselect on; the assessment of what energies should be considered small should be addressed based on the energy distribution of the implemented state. Performing QPE on the initial state |*ψ*〉 *=* ∑*_{EϕE}* |*E*〉 and then postselecting on an observed value *xₒ*, one obtains the following unnormalized state [2]: $\frac{1}{{2}^{k}} {\sum }_{E} {φ}_{E} | E 〉 | {x}_{o} 〉 \left(\frac{1 - {e}^{2 πi {2}^{k} \left(E-{x}_{o}/{2}^{k}\right)}}{1 - {e}^{2 πi \left(E-{x}_{o}/{2}^{k}\right)}}\right)$
meaning that the probability (= |coefficient|²) for energy *E* after the measurement becomes ${\left|{φ}_{E}\right|}^{2} \frac{1}{{2}^{2 k}} \left(\frac{{sin}^{2} \left(π{2}^{k}E\right)}{{sin}^{2} \left(π\left[E-{x}_{o}/{2}^{k}\right]\right)}\right) .$

It is assumed that the standard deviation of the energy distribution of the initial state is small compared to the width of the spectrum of the Hamiltonian, which allows one to approximate the denominator in the above factor by Taylor expansion; one can see that the weight after measurement is suppressed as the distance $d = {min}_{n} \left|{2}^{k}\left(E+n\right)-{x}_{o}\right|$ from the measurement outcome is increased, scaling with inverse squared distance: $∼ \frac{1}{{d}^{2}}$. It is noted that the minimization over *n* in the definition of the distance is used as the expanded denominator is a periodic function.

If the precision of the coarse QPE and the postselection values are chosen appropriately, the high-energy weight of the distribution is suppressed.

### QETU filtering

Using QETU [16] (other Hamiltonian polynomial-based methods [17, 18] will work similarly) a polynomial function of the Hamiltonian that retains low energies and filters high energies is implemented.

In short, the method consists of a quantum signal processing circuit [19, 20] that implements a unitary matrix that block encodes a polynomial function *f*(*H*) = *P*(cos(H/2)), where *H* is the Hamiltonian of interest and *P* is an even polynomial of degree d. A scheme of the quantum circuit is shown in the schematic below. The circuit works by implementing *U* = *e*^{-*iH*} and its Hermitian conjugate controlled on a single ancilla a total number of *d* times.

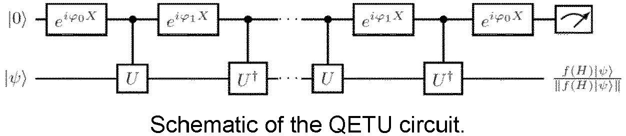

The parameters *ϕ*₀, *ϕ*₁, ..., *ϕ*_{*d*/2} are determined based on the polynomial of interest. Upon measuring the ancilla qubit at the end and obtaining the outcome 0, the implementation has been successful. The probability of success is given by || *P*(cos(*H*/2)) |*ψ*〉 ||.

The polynomial that needs to be implemented for the energy filtering task should be a symmetric function that retains low energies and filters high energies. In particular, the spectrum of the Hamiltonian is chosen to lie within the interval [-*π* + *η*, 0 - *η*]. If necessary, this can be done by adding a constant to and/or rescaling the Hamiltonian before performing QETU. It is noted that this is contrary to the original setting of Ref. [16] (the spectrum is contained in [*η,π*- *η*]) and coarse QPE mentioned above; the reason for this change is better performance as will become clear below. With this, low energies will be close to -*π* and thus cos(*H*/2) will be close to zero. As the goal is to retain low energies and filter out high energies, a polynomial P whose value is close to 1 when its argument is close to 0, and close to 0 when its argument is close to 1, is needed. First, the following combination of error functions is used to this end: ${ξ}_{k , μ} \left(x\right) = \frac{1}{2} \left[erf\left(-k\left(x-μ\right)\right)+erf\left(k\left(x+μ\right)\right)\right]$ with 0 < k and 0 < *µ* < 1 determining the steepness and position of the transitions in the function. The prescription in Appendix A of Ref. [21] is used to reconstruct the error function erf(kx) in terms of Chebyshev polynomials as follows: ${p}_{erf , k , n} = \frac{2 {ke}^{- {k}^{2} / 2}}{\sqrt{π}} \left({I}_{0}\left(\frac{{k}^{2}}{2}\right)+{\sum }_{j = 1}^{\left(n-1\right) / 2} {\left(-1\right)}^{j} {I}_{j} \left({k}^{2}/2\right)\left[\frac{{T}_{2 j + 1} \left(x\right)}{2 j + 1}-\frac{{T}_{2 j - 1} \left(x\right)}{2 j - 1}\right]\right)$ where *Tⱼ* is the degree *j* Chebyshev polynomial and *Iⱼ* is the modified Bessel function of the first kind. It is noted that *p*_{erf*,k,n*} is an odd polynomial of degree *n*; it is the degree *n* that controls the error in approximating erf(kx) and thus ensures that low energies are retained and high energies are filtered, and it should therefore be chosen to be large enough.

Applying a successful round of QETU filtering to a state |*ψ*〉 *=* ∑*_{EϕE}*|*E*〉 results in the following unnormalized state: ${\sum }_{E} {φ}_{E} P \left(cos\left(E/2\right)\right) | E 〉 | 0 〉$

This shows that, supposing one wishes to keep energies below *Eₗ* and filter energies above *Eᵤ*, one can choose a filtering function with $μ = \frac{cos \left({E}_{u}/2\right) + cos \left({E}_{l}/2\right)}{2}$ and $\frac{1}{k} =$ $ζ \frac{cos \left({E}_{u}/2\right) - cos \left({E}_{l}/2\right)}{2}$. The factor ζ is added so that it is possible to control the intensity of filtering while keeping the degree of the polynomial and the cost down.

The degree of the polynomial that needs to be used for this task will have a scaling of *O*(Γ⁻¹log*∈*⁻¹), where *∈* is the error in the polynomial approximation and Γ is the length scale over which the transition in the error functions in the equation for *ξ_{k,µ}*(*x*) happens, and thus should scale as *1*/*k.* In practice, *n* can be chosen by examining how good of an approximation is achieved for degree *n*.

### Costs of the above methods

To illustrate the advantages of the above methods (coarse QPE and QETU filtering) a simple analysis of the asymptotic cost is provided below by the following treatment: to suppress a high degree of weight at unwanted energies and keep low-energy weight mostly unsuppressed, one needs to differentiate energies at the level of the standard deviation of the energy distribution function of the initial state, which is shown as σ*_{E}.* The above two scenarios are considered separately.

In a coarse QPE setting, one needs the resolution to be of the order of *σ_{E}* at least, so that the unwanted weights are dropped when a low value outcome is postselected. This means that a number of queries to *e^{-iH}* is needed that scales as 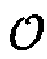 (1/σ_{E}).

On the other hand, in a QETU setting, again a polynomial is needed that discerns energies that are both close to -*π* but differ by a value close to 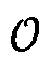 (σ_{E}). After taking cos(*H*/2), one can use a Taylor expansion to see that the resolution is still 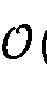 (σ_{E}) and thus a polynomial that transitions in a length scale scaling as 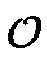 (σ_{E}) is needed. This causes the QETU approach to also have a scaling as 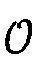 (1/σ_{E}). It is noted that if the spectrum had been chosen to lie in [*η,π* - *η*], the scaling would have been 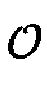 (1/σ²_{E}) as the required resolution after taking the cos function becomes .

As both of the above methods give the same 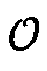 (1/σ_{E}) scaling, this should in principle be a subleading cost when compared to the cost of the most precise QPE (or other energy estimation), which would have a scaling 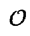 (1/ε), as the precision *∈* is expected to be considerably smaller than the width of the distribution given by *σ_{E}*.

It can be seen that the costs are asymptotically the same.

### Other aspects

Further, in a second aspect, in particular a method for preparing a quantum state for quantum energy estimation on a quantum chemical system is provided, the method comprising:
- classically computing states close to the ground state of the quantum chemical system,
- assessing the quality of the classically computed states based on the energy distribution of each state,
- selecting a state based on the assessment result, and
- implementing the selected state on a quantum computer.

The advantages and details of the second aspect are the same as the ones that are described for the respective features above and below.

In one embodiment of the second aspect, implementing the selected state comprises preparing and using a compressed representation of the sum of Slater determinants. The advantages and details of this embodiment of the second aspect are the same as the ones that are described for the respective features above and below.

In one embodiment of the second aspect, the method further comprises quantum refining of the implemented state on the quantum computer by filtering out higher energy contributions after state implementation. The advantages and details of this embodiment of the second aspect are the same as the ones that are described for the respective features above and below.

In addition, in a third aspect, in particular a method for preparing a quantum state for quantum energy estimation on a quantum chemical system is provided, the method comprising:
- classically computing a state close to the ground state of the quantum chemical system, and
- implementing the state on a quantum computer by preparing and using a compressed representation of the sum of Slater determinants.

The advantages and details of the third aspect are the same as the ones that are described for the respective features above and below.

In one embodiment of the third aspect, classically computing the state comprises classically computing a plurality of states close to the ground state of the quantum chemical system, assessing the quality of the classically computed states based on the energy distribution of each state, and selecting a state based on the assessment result for implementing. The advantages and details of this embodiment of the third aspect are the same as the ones that are described for the respective features above and below.

In one embodiment of the third aspect, the method further comprises quantum refining of the implemented state on the quantum computer by filtering out higher energy contributions after state implementation. The advantages and details of this embodiment of the third aspect are the same as the ones that are described for the respective features above and below.

Then, in a fourth aspect, in particular a method for preparing a quantum state for quantum energy estimation on a quantum chemical system is provided, the method comprising:
- classically computing a state close to the ground state of the quantum chemical system,
- implementing the state on a quantum computer, and
- refining of the implemented state on the quantum computer by filtering out higher energy contributions after state implementation.

The advantages and details of the fourth aspect are the same as the ones that are described for the respective features above and below.

In one embodiment of the fourth aspect, classically computing the state comprises classically computing a plurality of states close to the ground state of the quantum chemical system, assessing the quality of the classically computed states based on the energy distribution of each state, and selecting a state based on the assessment result for implementing. The advantages and details of this embodiment of the fourth aspect are the same as the ones that are described for the respective features above and below.

In one embodiment of the fourth aspect, preparing the state comprises preparing and using a compressed representation of the sum of Slater determinants. The advantages and details of this embodiment of the fourth aspect are the same as the ones that are described for the respective features above and below.

In one embodiment, the method further comprises categorizing the ground state energy estimation problem of the quantum chemical system with regard to quantum computational difficulty as either an easy problem, an intermediate problem, or a hard problem, and only in case the result shows an intermediate problem, performing all method steps. This allows the method to be used only in case specific conditions are met, i.e., in case the method is advantageous in terms of cost (computational/state preparation cost) relative to the classical method costs. The idea behind this is that with a given budget for state preparation one can have easy, intermediate, and hard problems. An easy problem is, in particular, one in which a very high-quality initial state with large support on the low-energy subspace of the Hamiltonian is possible to prepare on a quantum computer; on the other hand, a hard problem is one in which, with a given budget, it is only possible to prepare a poor-quality state with negligible weight over low energies of the Hamiltonian. It is noted that these depend on the given budget for state preparation. Now, one can also consider problems of intermediate hardness, where there is some non-negligible, but also not close to 1, weight over low-energy parts of the spectrum.

In particular, for a problem of the intermediate type, it is possible to obtain quantum advantage in quantum ground state energy determination over classical computational methods. This is because, by definition, for hard problems one cannot perform quantum routines effectively, and for easy problems it is very likely to find a good classical energy estimate which is hard/futile to beat quantumly.

In one embodiment, the method further comprises calculating, based on the energy distribution, the mean value of the distribution of the smallest energy obtained from within a quantum phase estimation budget of *N* outcomes, assessing whether it is below a target classical energy, and only if this is the case, performing all method steps. In particular, this provides a criterion for deciding if the method should be performed or not. The embodiment and the criterion are illustrated in the following. For this, the energy distribution is needed. Let the best classically achieved energy be denoted by *E_{T}*. Also, assuming one has access to the energy distribution of the initial state, one can calculate the distribution of best energy achievable through *N* QPE measurements within the budget and evaluate its mean value. Now, if the mean value is below the classical target energy *E_{T}*, this suggests the possibility for quantum advantage. In particular, there is possibility for improving the ground energy estimate using QPE.

The invention is explained in further detail below on the basis of preferred exemplary embodiments with reference to the drawings. In the drawings:
- Fig. 1: shows a schematic diagram to illustrate embodiments of the method for estimating a ground state energy of a quantum chemical system;
- Fig. 2: shows a schematic diagram to illustrate embodiments of the method for preparing a quantum state for quantum energy estimation on a quantum chemical system according to the second aspect;
- Fig. 3: shows a schematic diagram to illustrate embodiments of the method for preparing a quantum state for quantum energy estimation on a quantum chemical system according to the third aspect;
- Fig. 4: shows a schematic diagram to illustrate embodiments of the method for preparing a quantum state for quantum energy estimation on a quantum chemical system according to the fourth aspect;
- Fig. 5: shows a schematic of an embodiment of the system.

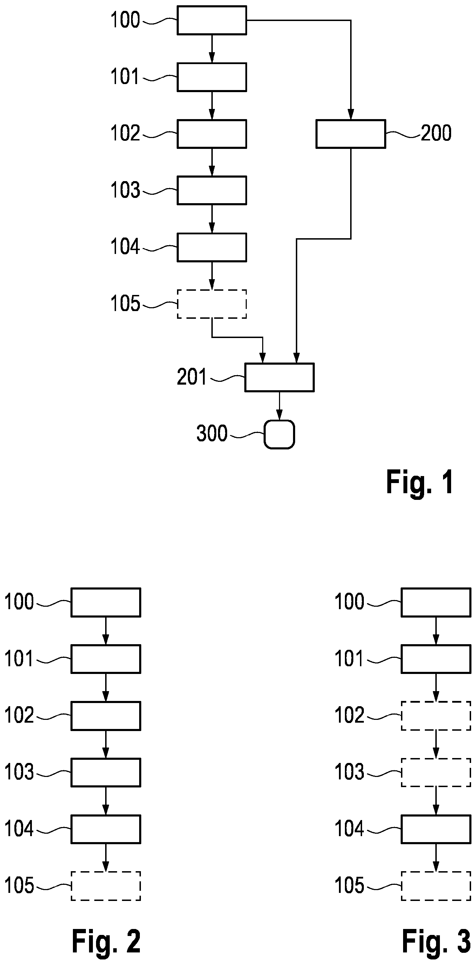

Fig. 1 shows a schematic diagram to illustrate embodiments of the method for estimating a ground state energy of a quantum chemical system.

In box 100 the Hamiltonian governing the physics of the quantum chemical system for which the ground state estimation should be performed is specified. There are a variety of systems of interest that may be considered, e.g., molecules, materials, effective spin models, etc. A variety of representations (e.g., first/second quantization) and approximations (e.g., pseudopotentials, rank factorization, quantum embedding, etc.) may be utilized to obtain the most suitable and efficient form of the Hamiltonian.

In box 101, with the Hamiltonian as the input, a classical computation is performed to obtain states close to the ground state of the quantum chemical system. The resulting states are a set of candidate states. In particular, the goal is to obtain an initial state that potentially has large support on the low-energy subspace of the Hamiltonian (e.g., the ground state). High overlap, for which high support is a proxy, is commonly a strict requirement for quantum algorithms to yield good estimates of the ground state energy. Different classical methods may be used: Hartree-Fock, Coupled Cluster (CC), selective configuration interaction methods (such as stochastic heat bath configuration space, SHCI), and density matrix renormalization group (DMRG).

In box 102 the quality of the classically computed states is assessed based on the energy distribution of each state. In particular, the different classical solutions (candidate states) are assessed based on an approach that relies on approximating the overlaps of the wavefunction with the Hamiltonian's true eigenstates, i.e., obtaining an approximate energy distribution of the state. This provides a means for quality comparison between different potential states.

Box 102 may comprise approximating the energy distributions of at least a subset of the classically computed states through an Edgeworth series expansion using the Hamiltonian moments of the state and/or by the resolvent (matrix product state-based) method. The details for these methods are given in the general description of this disclosure.

Alternatively or additionally, box 102 may further comprise approximating the energy distributions of at least a subset of the classically computed states through coarse quantum phase estimation. The details for these methods are given in the general description of this disclosure. It is noted that the classically computed states must be implemented on a quantum computer in order to perform coarse QPE.

In box 103, in particular a state of the candidate states is selected based on the assessment result. In particular, to choose a state from the candidate states as a solution for the initial state to start with, the support on the low-energy subspace is an important quantity to consider as this is proportional to the probability of obtaining energy estimates as low as those energies in a quantum routine. In particular, a state that has the largest weights for smaller energies is chosen as the best candidate. The details are provided in the general description.

In box 104 the selected state is implemented on a quantum computer. In particular, the classical state is encoded on the quantum computer. In other words, the selected state is encoded in a quantum register of the quantum computer.

In particular, implementing the selected state in box 104 may comprise preparing and using a compressed representation of the sum of Slater determinants. The details for these methods are given in the general description of this disclosure.

Alternatively, implementing the selected state in box 104 may comprise preparing and using a matrix product state form. The details for these methods are given in the general description of this disclosure.

In an optional box 105 quantum refining of the implemented state on the quantum computer is performed by filtering out higher energy contributions after state implementation. The details for these methods are given in the general description of this disclosure.

Quantum refining in box 105 may comprise using at least one of: Hamiltonian polynomial methods, coarse quantum phase estimation, quantum imaginary time evolution, and in particular quantum eigenvalue transformation of unitary matrices with real polynomials (QETU). The details for these methods are given in the general description of this disclosure.

In box 200 the Hamiltonian of the system is encoded into the quantum computer. In particular, this is done by linear combination of unitaries (LCU) which may further be used for the qubitization of the system. In qubitization, a unitary operator *Q* (called the qubitization of *H*) is formed. Roughly speaking, the eigenstates of *H* are also eigenstates of *Q*, with eigenvalues related to those of *H*. Other methods, such as Hamiltonian simulation, may also be used.

In box 201 the ground state energy of the quantum chemical system is estimated on the quantum computer based on the implemented state. To obtain the ground state energy estimate, there are many methods that may be used; generically these methods need an initial state input that has a high enough support on the low-energy subspace of the system Hamiltonian. This state was derived in boxes 100 to 105. As the methods described in this disclosure provide an initial state with large overlap, the methods are applicable generally. In particular, quantum phase estimation (QPE) is used for estimating the energy. The overlap between an initial state and a particular state of interest determines the number of times QPE measurements need to be performed as 1/overlap². Thus, better overlap, achieved by the embodiments of the method described in this disclosure, directly decreases the quantum computational cost.

The final result 300 is an improved estimate of the ground state energy of the Hamiltonian of the system.

Fig. 2 shows a schematic diagram to illustrate embodiments of the method for preparing a quantum state for quantum energy estimation on a quantum chemical system according to the second aspect.

In box 100 the Hamiltonian governing the physics of the quantum chemical system for which the ground state estimation should be performed is specified. There are a variety of systems of interest that may be considered, e.g., molecules, materials, effective spin models, etc. A variety of representations (e.g., first/second quantization) and approximations (e.g., pseudopotentials, rank factorization, quantum embedding, etc.) may be utilized to obtain the most suitable and efficient form of the Hamiltonian.

In box 101, with the Hamiltonian as the input, a classical computation is performed to obtain states close to the ground state of the quantum chemical system. The resulting states are a set of candidate states. In particular, the goal is to obtain an initial state that potentially has a large overlap with the lowest-energy eigenstates of the Hamiltonian (e.g., the ground state). High overlap, for which support is a proxy, is commonly a strict requirement for quantum algorithms to yield good estimates of the ground state energy. Different classical methods may be used: Hartree-Fock, Coupled Cluster (CC), selective configuration interaction methods (such as stochastic heat bath configuration space, SHCI), and density matrix renormalization group (DMRG).

In box 102 the quality of the classically computed states is assessed based on the energy distribution of each state. In particular, the different classical solutions (candidate states) are assessed based on an approach that relies on approximating the overlaps of the wavefunction with the Hamiltonian's true eigenstates, i.e., obtaining an approximate energy distribution of the state. This provides a means for quality comparison between different potential states.

Box 102 may comprise approximating the energy distributions of at least a subset of the classically computed states through an Edgeworth series expansion using the Hamiltonian moments of the state and/or by the resolvent (matrix product state-based) method. The details for these methods are given in the general description of this disclosure.

Alternatively or additionally, box 102 may further comprise approximating the energy distributions of at least a subset of the classically computed states through coarse quantum phase estimation. The details for these methods are given in the general description of this disclosure. It is noted that the classically computed states must be implemented on a quantum computer in order to perform coarse QPE.

In box 103, in particular a state of the candidate states is selected based on the assessment result. In particular, to choose a state from the candidate states as a solution for the initial state to start with, the support on the low-energy subspace is an important quantity to consider as this is proportional to the probability of obtaining energy estimates as low as those energies in a quantum routine. In particular, a state that has the largest weights for smaller energies is chosen as the best candidate. The details are provided in the general description.

In box 104 the selected state is implemented on a quantum computer. In particular, the classical state is encoded on the quantum computer. In other words, the selected state is encoded in a quantum register of the quantum computer.

In particular, implementing the selected state in box 104 may comprise preparing and using a compressed representation of the sum of Slater determinants. The details for these methods are given in the general description of this disclosure.

Alternatively, implementing the selected state in box 104 may comprise preparing and using a matrix product state form. The details for these methods are given in the general description of this disclosure.

In an optional box 105 quantum refining of the implemented state on the quantum computer is performed by filtering out higher energy contributions after state implementation. The details for these methods are given in the general description of this disclosure.

Quantum refining in box 105 may comprise using at least one of: Hamiltonian polynomial methods, coarse quantum phase estimation, quantum imaginary time evolution, and in particular quantum eigenvalue transformation of unitary matrices with real polynomials (QETU). The details for these methods are given in the general description of this disclosure.

Fig. 3 shows a schematic diagram to illustrate embodiments of the method for preparing a quantum state for quantum energy estimation on a quantum chemical system according to the third aspect.

In box 100 the Hamiltonian governing the physics of the quantum chemical system for which the ground state estimation should be performed is specified. There are a variety of systems of interest that may be considered, e.g., molecules, materials, effective spin models, etc. A variety of representations (e.g., first/second quantization) and approximations (e.g., pseudopotentials, rank factorization, quantum embedding, etc.) may be utilized to obtain the most suitable and efficient form of the Hamiltonian.

In box 101, with the Hamiltonian as the input, a classical computation is performed to obtain a state close to the ground state of the quantum chemical system. In particular, the goal is to obtain an initial state that potentially has a large overlap with the lowest-energy eigenstates of the Hamiltonian (e.g., the ground state). High overlap, for which support is a proxy, is commonly a strict requirement for quantum algorithms to yield good estimates of the ground state energy. Different classical methods may be used: Hartree-Fock, Coupled Cluster (CC), selective configuration interaction methods (such as stochastic heat bath configuration space, SHCI), and density matrix renormalization group (DMRG).

Optionally, in box 101, classically computing the state may comprise classically computing a plurality of states close to the ground state of the quantum chemical system.

In optional box 102 the quality of the classically computed states is assessed based on the energy distribution of each state. In particular, the different classical solutions (candidate states) are assessed based on an approach that relies on approximating the overlaps of the wavefunction with the Hamiltonian's true eigenstates, i.e., obtaining an approximate energy distribution of the state. This provides a means for quality comparison between different potential states.

Optional box 102 may comprise approximating the energy distributions of at least a subset of the classically computed states through an Edgeworth series expansion using the Hamiltonian moments of the state and/or by the resolvent (matrix product state-based) method. The details for these methods are given in the general description of this disclosure.

Alternatively or additionally, optional box 102 may further comprise approximating the energy distributions of at least a subset of the classically computed states through coarse quantum phase estimation. The details for these methods are given in the general description of this disclosure. It is noted that the classically computed states must be implemented on a quantum computer in order to perform coarse QPE.

In optional box 103, in particular a state of the candidate states is selected based on the assessment result. In particular, to choose a state from the candidate states as a solution for the initial state to start with, the support on the low-energy subspace is an important quantity to consider as this is proportional to the probability of obtaining energy estimates as low as those energies in a quantum routine. In particular, a state that has the largest weights for smaller energies is chosen as the best candidate. The details are provided in the general description.

In box 104 the (optionally selected) state is implemented on a quantum computer. In particular, the classical state is encoded on the quantum computer. In other words, the selected state is encoded in a quantum register of the quantum computer.

Implementing the selected state in box 104 comprises preparing and using a compressed representation of the sum of Slater determinants. The details for these methods are given in the general description of this disclosure.

In an optional box 105 quantum refining of the implemented state on the quantum computer is performed by filtering out higher energy contributions after state implementation. The details for these methods are given in the general description of this disclosure.

Quantum refining in box 105 may comprise using at least one of: Hamiltonian polynomial methods, coarse quantum phase estimation, quantum imaginary time evolution, and in particular quantum eigenvalue transformation of unitary matrices with real polynomials (QETU). The details for these methods are given in the general description of this disclosure.

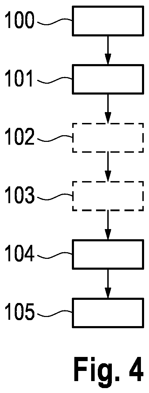

Fig. 4 shows a schematic diagram to illustrate embodiments of the method for preparing a quantum state for quantum energy estimation on a quantum chemical system according to the fourth aspect.

In box 100 the Hamiltonian governing the physics of the quantum chemical system for which the ground state estimation should be performed is specified. There are a variety of systems of interest that may be considered, e.g., molecules, materials, effective spin models, etc. A variety of representations (e.g., first/second quantization) and approximations (e.g., pseudopotentials, rank factorization, quantum embedding, etc.) may be utilized to obtain the most suitable and efficient form of the Hamiltonian.

In box 101, with the Hamiltonian as the input, a classical computation is performed to obtain a state close to the ground state of the quantum chemical system. In particular, the goal is to obtain an initial state that potentially has a large overlap with the lowest-energy eigenstates of the Hamiltonian (e.g., the ground state). High overlap, for which support is a proxy, is commonly a strict requirement for quantum algorithms to yield good estimates of the ground state energy. Different classical methods may be used: Hartree-Fock, Coupled Cluster (CC), selective configuration interaction methods (such as stochastic heat bath configuration space, SHCI), and density matrix renormalization group (DMRG). Optionally, in box 101, classically computing the state may comprise classically computing a plurality of states close to the ground state of the quantum chemical system.

In optional box 102 the quality of the classically computed states is assessed based on the energy distribution of each state. In particular, the different classical solutions (candidate states) are assessed based on an approach that relies on approximating the overlaps of the wavefunction with the Hamiltonian's true eigenstates, i.e., obtaining an approximate energy distribution of the state. This provides a means for quality comparison between different potential states.

Optional box 102 may comprise approximating the energy distributions of at least a subset of the classically computed states through an Edgeworth series expansion using the Hamiltonian moments of the state and/or by the resolvent (matrix product state-based) method. The details for these methods are given in the general description of this disclosure.

Alternatively or additionally, optional box 102 may further comprise approximating the energy distributions of at least a subset of the classically computed states through coarse quantum phase estimation. The details for these methods are given in the general description of this disclosure. It is noted that the classically computed states must be implemented on a quantum computer in order to perform coarse QPE.

In optional box 103, in particular a state of the candidate states is selected based on the assessment result. In particular, to choose a state from the candidate states as a solution for the initial state to start with, the support on the low-energy subspace is an important quantity to consider as this is proportional to the probability of obtaining energy estimates as low as those energies in a quantum routine. In particular, a state that has the largest weights for smaller energies is chosen as the best candidate. The details are provided in the general description.

In box 104 the (optionally selected) state is implemented on a quantum computer. In particular, the classical state is encoded on the quantum computer. In other words, the selected state is encoded in a quantum register of the quantum computer.

In particular, implementing the selected state in box 104 may comprise preparing and using a compressed representation of the sum of Slater determinants. The details for these methods are given in the general description of this disclosure.

Alternatively, implementing the selected state in box 104 may comprise preparing and using a matrix product state form. The details for these methods are given in the general description of this disclosure.

In box 105 quantum refining of the implemented state on the quantum computer is performed by filtering out higher energy contributions after state implementation. The details for these methods are given in the general description of this disclosure.

Quantum refining in box 105 may comprise using at least one of: Hamiltonian polynomial methods, coarse quantum phase estimation, quantum imaginary time evolution, and quantum eigenvalue transformation of unitary matrices with real polynomials (QETU). The details for these methods are given in the general description of this disclosure.

The methods shown in Figures 2, 3, and 4 may all be used within a method for estimating a ground state energy of a quantum chemical system as shown in Fig. 1 and described above. In particular, every individual feature of the embodiments in this disclosure may be combined with other features in any combination. In particular, every variant of a combination of at least one of assessing the quality, implementing the state by preparing and using a compressed representation of Slater determinants, and quantum refining may be used.

Any of the methods described may further comprise categorizing the ground state energy estimation problem of the quantum chemical system with regard to computational difficulty as either an easy problem, an intermediate problem, or a hard problem, and only in case the result shows an intermediate problem, performing all method steps.

Any of the methods described may further comprise calculating, based on the energy distribution, the mean value of the distribution of the smallest energy obtained from within a quantum phase estimation budget of N outcomes, assessing whether it is below a target classical energy, and only if this is the case, performing all method steps.

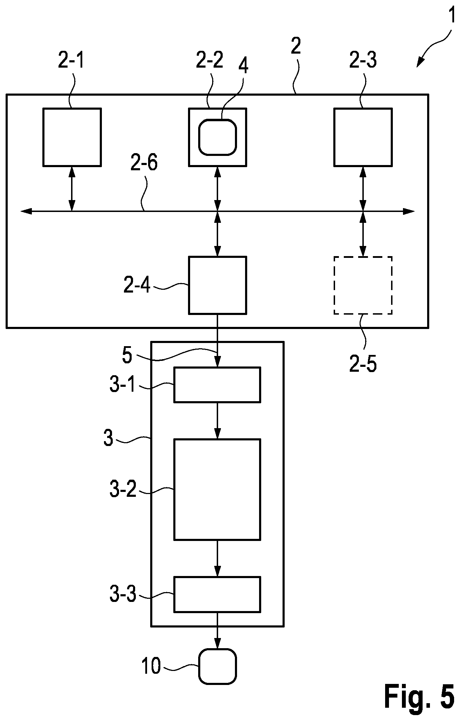

Fig. 5 shows a schematic of an embodiment of the system 1. The system 1 comprises a classical computer 2 and a quantum computer 3.

The classical computer 2 comprises at least one processor 2-1, at least one memory 2-2, a persistent storage 2-3, a communication interface 2-4, and optionally also a display 2-5. These components 2-1, 2-2, 2-3, 2-4, 2-5 are connected via at least one communication bus 2-6. The classical computer is configured to perform the classical computations of a method according to any one of the embodiments described above.

The quantum computer 3 comprises at least one input quantum register 3-1, a quantum circuit 3-2, and an output register 3-3. The quantum circuit 3-2 comprises quantum gates which act upon a state that is input to the input quantum register 3-1. The quantum computer 3 is configured to perform the quantum computations of the embodiment. To this end, the initial state derived by the classical computation is implemented in the quantum input register 3-1 and afterwards propagated through the quantum circuit 3-2. The output register 3-3 then holds the result. To obtain the result, in particular, a number of QPE measurements are necessary. In the last phase of the QPE, the quantum computer 3 performs measurements on the qubits to extract the desired information. This process is, in particular, repeated several times to produce the chemically accurate prediction of the ground state energy.

The quantum computer 3 may be composed of superconducting qubits, photonic qubits, trapped-ion qubits, silicon-based qubits, and/or neutral atoms.

The methods and system described in this disclosure decrease the cost of energy estimation, in particular in the QPE algorithm. They decrease the number of times that the final most accurate and most costly QPE needs to be performed by preparing a state with larger support on the low-energy subspace of the Hamiltonian. By increasing the overlap, there is also the potential to make each QPE measurement less costly; in some cases, due to a poor overlap, the total QPE evolution time might need to be as long as 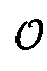 (1/overlap²); with a better overlap, achievable by using the methods and the system described in this disclosure, the total time can be shortened and can essentially be independent of the overlap. This is, in particular, due to preventing leakage from high-energy states and thus decreasing the total evolution time required for each QPE round.

### Appendix A

In general, the Edgeworth series terms can be written as [12]: ${p}_{E} \left(x\right) = \frac{{e}^{- {x}^{2} / 2}}{\sqrt{2 π}} \left[1+{\sum }_{s = 1}^{∞} {\sum }_{\left\{{k}_{m}\right\}} {He}_{s + 2 r} \left(x\right) {\prod }_{m = 1}^{s} \frac{1}{{k}_{m} !} {\left(\frac{{κ}_{m + 2}}{\left(m+2\right) !}\right)}^{{k}_{m}}\right]$ where the summation over {*kₘ*} in the above denotes summation over all non-negative integer solutions of the Diophantine equation ${k}_{1} + 2 {k}_{2} + ⋯ + {sk}_{s} = s$ and r is the sum of these integers for each solution: *r* = ∑*kₘ*.

### Appendix B

### MPS to sum of Slater determinants

The goal is to calculate the largest coefficients $c \left({n}_{1},…,{n}_{N}\right) = {\sum }_{{α}_{1} … {α}_{N - 1}} {A}_{1 ; {α}_{1}}^{{n}_{1}} … {A}_{N ; {α}_{N - 1}}^{{n}_{N}}$ in a sum of Slater determinants (SOS) expansion. Based on Appendix A of Ref. [7], one starts from a left canonical form and sets a threshold for keeping terms in the SOS. Partial coefficients such as ${c}_{{α}_{p}}^{\left(p\right)} \left({n}_{1},…,{n}_{p}\right) = {\sum }_{{α}_{1} … {α}_{p - 1}} {A}_{1 ; {α}_{1}}^{{n}_{1}} … {A}_{p ; {α}_{p - 1} {α}_{p}}^{{n}_{p}}$ are formed and whenever a norm of the partial coefficient ${\sum }_{{α}_{p}} {\left|{c}_{{α}_{p}}^{\left(p\right)}\left({n}_{1},…,{n}_{p}\right)\right|}^{2}$ goes below a threshold, all Slater determinants with the prefix (*n*₁, ..., *nₚ*), i.e., of the form |*n*_{1,} ..., *nₚ*,...〉, are dropped from the SOS. This way, owing to the left canonical form of the MPS, it is ensured that all the terms with coefficients above the threshold are recovered in the SOS.

### Sum of Slater determinants to MPS

For this task, one starts with a bond dimension 1 MPS that corresponds to the largest coefficient Slater determinant in the SOS (could be the Hartree-Fock state or not); one also makes an auxiliary copy of it. Using matrix product operators consisting of a number of *c*^{†} and c operators, the auxiliary bond dimension 1 MPS is transformed to the Slater determinant with the second largest coefficient. The new auxiliary MPS is added to the main MPS and the procedure goes on until all coefficients are added. It is noted that the auxiliary MPS remains bond dimension 1 while the bond dimension of the main MPS grows, and one can compress the main MPS as more and more terms are added to it.

### References

[1] A. Y. Kitaev, arXiv preprint quant-ph/9511026 (1995).
[2] M. A. Nielsen and I. L. Chuang, Quantum computation and quantum information (Cambridge university press, 2010).
[3] N. M. Tubman, C. Mejuto-Zaera, J. M. Epstein, D. Hait, D. S. Levine, W. Huggins, Z. Jiang, J. R. McClean, R. Babbush, M. Head-Gordon, et al., arXiv preprint arXiv:1809.05523 (2018).
[4] C. Schön, E. Solano, F. Verstraete, J. I. Cirac, and M. M. Wolf, Physical review letters 95, 110503 (2005).
[5] D. Malz, G. Styliaris, Z.-Y. Wei, and J. I. Cirac, arXiv preprint arXiv:2307.01696 (2023).
[6] H. Zhai and G. K. Chan, The Journal of Chemical Physics 154 (2021).
[7] S. Lee, H. Zhai, S. Sharma, C. J. Umrigar, and G. K.-L. Chan, Journal of Chemical Theory and Computation 17, 3414 (2021).
[8] G. H. Low, V. Kliuchnikov, and L. Schaeffer, arXiv preprint arXiv:1812.00954 (2018).
[9] S.-J. Ran, Physical Review A 101, 032310 (2020).
[10] M. S. Rudolph, J. Chen, J. Miller, A. Acharya, and A. Perdomo-Ortiz, arXiv preprint arXiv:2209.00595 (2022).
[11] M. B. Dov, D. Shnaiderov, A. Makmal, and E. G. D. Torre, arXiv preprint arXiv:2207.00028 (2022).
[12] S. Blinnikov and R. Moessner, Astronomy and Astrophysics Supplement Series 130, 193 (1998).
[13] E. Jeckelmann, Physical Review B 66, 045114 (2002).
[14] E. Ronca, Z. Li, C. A. Jimenez-Hoyos, and G. K.-L. Chan, Journal of chemical theory and computation 13, 5560 (2017).
[15] M. Wand and M. Jones, Kernel Smoothing, Chapman & Hall/CRC Monographs on Statistics & Applied Probability (60) (Chapman & Hall/CRC, 1994).
[16] Y. Dong, L. Lin, and Y. Tong, PRX Quantum 3, 040305 (2022).
[17] L. Lin and Y. Tong, Quantum 4, 372 (2020).
[18] Y. Ge, J. Tura, and J. I. Cirac, Journal of Mathematical Physics 60 (2019).
[19] G. H. Low and I. L. Chuang, Physical review letters 118, 010501 (2017).
[20] J. M. Martyn, Z. M. Rossi, A. K. Tan, and I. L. Chuang, PRX Quantum 2, 040203 (2021).
[21] G. H. Low and I. L. Chuang, arXiv preprint arXiv:1707.05391 (2017).
[22] L. Lin and Y. Tong, PRX Quantum 3, 010318 (2022).
[23] Y. Ge, J. Tura, and J. I. Chirac, Journal of Mathematical Physics 60 (2019).

### Reference numerals

- 1: system
- 2: classical computer
- 2-1: processor
- 2-2: memory
- 2-3: persistent storage
- 2-4: communication interface
- 2-5: display
- 2-6: communication bus
- 3: quantum computer
- 3-1: input quantum register
- 3-2: quantum circuit
- 3-3: output register
- 4: state
- 5: selected/implemented state
- 100-105: method boxes
- 200-201: method boxes
- 300: final result

## Claims

1. Method for estimating a ground state energy (10) of a quantum chemical system, the method comprising:
- classically computing states (4) close to the ground state of the quantum chemical system,
- assessing the quality of the classically computed states (4) based on the energy distribution of each state (4),
- selecting a state (4) based on the assessment result,
- implementing the selected state (5) on a quantum computer (3), and
- estimating the ground state energy (10) of the quantum chemical system on the quantum computer based on the implemented state (5).

2. Method according to claim 1, **characterized in that** the method further comprises approximating the energy distributions of at least a subset of the classically computed states (4) through an Edgeworth series expansion using the Hamiltonian moments of the state and/or by the resolvent (matrix product state-based) method.

3. Method according to claim 1 or 2, **characterized in that** the method further comprises approximating the energy distributions of at least a subset of the classically computed states (4) through coarse quantum phase estimation.

4. Method according to any one of the preceding claims, **characterized in that** implementing the selected state (5) comprises preparing and using a compressed representation of the sum of Slater determinants.

5. Method according to any one of claims 1 to 3, **characterized in that** implementing the selected state (5) comprises preparing and using a matrix product state form.

6. Method according to any one of the preceding claims, **characterized in that** the method further comprises quantum refining of the implemented state (5) on the quantum computer (3) by filtering out higher energy contributions after state implementation.

7. Method according to claim 6, **characterized in that** quantum refining comprises using at least one of: Hamiltonian polynomial methods, coarse quantum phase estimation, and quantum imaginary time evolution.

8. Method for preparing a quantum state (4,5) for quantum energy estimation on a quantum chemical system, the method comprising:
- classically computing states (4) close to the ground state of the quantum chemical system,
- assessing the quality of the classically computed states (4) based on the energy distribution of each state (4),
- selecting a state (4) based on the assessment result, and
- implementing the selected state (5) on a quantum computer (3).

9. Method according to claim 8, **characterized in that** preparing the selected state (5) comprises preparing and using a compressed representation of the sum of Slater determinants.

10. Method according to claim 8 or 9, **characterized in that** the method further comprises quantum refining of the implemented state (5) on the quantum computer (3) by filtering out higher energy contributions after state implementation.

11. Method for preparing a quantum state (4,5) for quantum energy estimation on a quantum chemical system, the method comprising:
- classically computing a state (4) close to the ground state of the quantum chemical system, and
- implementing the state (4) on a quantum computer (3) by preparing and using a compressed representation of the sum of Slater determinants.

12. Method for preparing a quantum state (4,5) for quantum energy estimation on a quantum chemical system, the method comprising:
- classically computing a state (4) close to the ground state of the quantum chemical system,
- implementing the state (4) on a quantum computer (3), and
- refining of the implemented state (5) on the quantum computer (3) by filtering out higher energy contributions after state implementation.

13. Method according to claim 12, **characterized in that** classically computing the state comprises classically computing a plurality of states (4) close to the ground state of the quantum chemical system, assessing the quality of the classically computed states (4) based on the energy distribution of each state (4), and selecting a state (4) based on the assessment result for implementing.

14. Method according to any one of the preceding claims, **characterized in that** the method further comprises categorizing the ground state energy estimation problem of the quantum chemical system with regard to computational difficulty as either an easy problem, an intermediate problem, or a hard problem, and only in case the result shows an intermediate problem, performing all method steps.

15. System (1), comprising:
- a classical computer (2), and
- a quantum computer (3),
wherein the classical computer (2) is configured to perform the classical computations of a method according to any one of the preceding claims, and wherein the quantum computer (3) is configured to perform the quantum computations of the method.
